# EUROPEAN PATENT APPLICATION

(11) **EP 1 985 635 A1**
(43) Date of publication of application: **29.10.2008**
(21) Application number: 07714164.6
(22) Date of filing: 14.02.2007
(51) Int. Cl.: C08B 37/00, A61K 36/00, A61P 1/16, A61P 3/04, A61P 3/06, A61P 19/06, A61P 37/08

(54) **PROCESS FOR PRODUCING POLYSACCHARIDE FROM COFFEE BEAN OR/AND COFFEE EXTRACTION RESIDUE**

(30) Priority: 14.02.2006 JP 2006036887
(71) Applicant: UCC Ueshima Coffee Co., Ltd., Kobe-shi, Hyogo 650-0015 (JP); Osaka Prefecture University Public Corporation, Osaka 599-8531 (JP)
(72) Inventor: IWAI, Kazuya, Takatsuki-shi, Osaka 569-0036 (JP); KASAI, Naoya, Sennan-gun, Osaka 590-0405 (JP)
(74) Representative: Müller-Boré & Partner Patentanwälte
(86) International application number: PCT/JP2007/052611
(87) International publication number: WO 2007/094360

(57) **Abstract**

A process for efficiently producing a polysaccharide from coffee beans or/and a coffee extraction residue. Use of these has not received attention because of difficulty in decomposing these. The process, which is for producing a polysaccharide from coffee beans or/and a coffee extraction residue, comprises: a step (a) in which the coffee beans or/and coffee extraction residue are reduced to particles having a diameter of 10.0 µm to 5.0 mm; a step (b1) in which after the step (a), the particles are heated at 50-100°C in the presence of a dilute alkali; a step (c1) in which after completion of the step (b1), cellulase is caused to act thereon; a step (b2) in which after the step (c1), a heat treatment is conducted at 120°C or higher in the presence of a dilute alkali; and a step (c2) in which after the step (b2), cellulase is caused to act thereon.

## Description

### FIELD OF THE INVENTION

The present invention relates to a method of producing polysaccharides from coffee beans or/and a coffee extraction residue, and particularly to a method of efficiently extracting/producing polysaccharides composed of arabinogalactan (galactan) and galactomannan from coffee beans or/and a coffee extraction residue. The term, "coffee beans" as used in the present invention mean raw coffee beans before roasting or roasted coffee beans subjected to a roasting treatment at high temperature thereby allowing the Maillard reaction to proceed, while the term "coffee extraction residue" means a residue after extracting a soluble solid from roasted coffee beans using such as water, warm water or steam.

### BACKGROUND ART

Now, coffee is luxury to be drunk, which is obtained by harvesting fruits from coffee trees and purifying the albumen portion, followed by roasting, milling and further extraction. Polysaccharides account for about 48% of the albumen portion and are composed of galactomannan, arabinogalactan and cellulose (see, for example, Non-Patent Document 1).

Galactomannans are polysaccharides composed of galactose and mannose, and examples of galactomannan distributing usually on the market include such as Fenugreek gum obtained from seeds of Fenugreek, guar gum obtained from seeds of guar, locust bean gum obtained from seeds of locust bean, tara gum obtained from seeds of tara and cassia gum obtained from seeds of cassia. Galactomannan can be produced by extracting the above plant with such as water, hot water, alkali or acid.

Galactomannans are polysaccarides which cannot be digested by human and are therefore said generally to be a dietary fiber. Galactomannans have a property of absorbing moisture to form a gel when dissolved in water, and are widely used as a thickener in industrial raw materials for foods, cosmetics, medical supplies and the like.

These galactomannans have such a feature that a ratio of galactose to mannose varies depending on the kind of plants from which raw materials are derived. For example, a ratio of galactose to mannose is 1:1 for Fenugreek gum, 1:2 for guar gum, and 1:4 for locust bean gum. Galactomannans have such a property that water solubility, emulsifiability and stability are improved as the ratio of galactose to mannose increases. The reason is that 100% mannan containing no galactose is insoluble in water, and galactose in the side chain enables a polymer of mannan to extend thereby preventing formation of a bound substance which is insoluble in water.

Therefore, it is said that galactomannans containing a larger amount of galasctose are useful in food, cosmetic and medical supply industries. A method of purification of Fenugreek gum containing the largest amount of galactose among galactomannans is disclosed (see, for example, Patent Document 1).

It has recently become apparent that galactomannans have various effects as a result of an intensive study on functionality of galactomannans other than the role as a thickener and a stabilizer in food, cosmetic and medical supply industries. Examples of the effect include effects such as reducing cholesterol in blood, improving and preventing an impaired liver function, suppressing absorption of a purine body, preventing obesity and improving allergic constitution. Therefore, galactomannans are functional food materials which will attract attention in future.

Galactomannans are also contained in coffee beans. It is considered that, if galactomannans with good quality are contained in plant materials such as coffee beans which are distributed/consumed in large quantities in the world, these plant materials are very useful as resources of galactomannans. It is said that a ratio of galactose to mannose is very small in galactomannans contained in coffee beans, and the ratio is from 1:16 to 1:18 for raw beans (see, for example, Non-Patent Document 2). These polysaccharides containing a large amount of mannose are slightly water soluble and are hardly used in the food industry or the like and, therefore, galactomannans derived from coffee beans have never been industrially used.

Arabinogalactan is a kind of water soluble hemicelluloses and is a cell wall constituent component which is common to all plants, and is a generic name of polysaccharides in which a side chain composed of arabinose or galactose is bonded to a main chain composed of galactose. Arabinogalactan is classified roughly into Type I arabinogalactan and Type II arabinogalactan according to a difference in structure. In the case of Type I arabinogalactan, a main chain having a β-(1→4)-galactopyranoside bond and a side chain having an α-L-arabonose residue are bonded. Type II arabinogalactan is made of a main chain having a β-(1→3)-galactopyranoside bond and a side chain composed of a β-(1→6)-galactopyranoside bond modified with α-L-arabonose, and has such a feature that it contains an ultratrace amount of xylose, rhamnose, fucose, glucuronic acid and the like.

A large amount of Type II arabinogalactan is contained in the albumen portion of coffee beans. Non-patent Document 1 discloses that the content of Type II arabinogalactan in robusta (Coffea canephora var. robusta) is 17% in coffee beans (dry weight basis) and is about 3% more than that of arabica (Coffea arabica).

When coffee beans are roasted, arabinogalactan is decomposed by heat upon roasting and arabinose in the side chain is cleaved thereby converting into an aroma component and a pigment component. Therefore, roasted coffee beans contain arabinogalactan containing a small amount of arabonose (see, for example, Non-Patent Document 3).

Since arabinogalactan has low viscosity among water soluble polysaccharides, the solid concentration can be increased without increasing viscosity. Arabinogalactan is used in ink for the purpose of improving color transferability, improving stability and the like. Recently, use of arabinogalactan as a functional food material is expected in view of a prebiotic effect (see, for example, Non-Patent Document 4).

Thus these coffee beans contain arabinogalactan and are considered to be useful in food, cosmetic and medical supply industries. However, it is difficult to extract arabinogalactan. It is necessary to extract with an alkali having very high concentration for a long time so as to extract arabinogalactan from coffee beans. For example, Non-Patent Document 5 describes that only a half amount of arabinogalactan is extracted even when extracted with 20% NaOH at 100°C for a whole day and night. As described above, since coffee beans contain a large amount of arabinogalactan, it was not practical for commercial production.

Patent Document 1: Japanese Unexamined Patent Publication No. 2005-341801
Non-Patent Document 1: Bradbury, A G W.; Halliday, D J. Chemical structures of coffee bean polysaccharide. J Agric Food Chem. 38, 389-392 (1990)
Non-Patent Document 2: R.J. Clarke, O.G. Vitzhum, COFFEE Recent Developments, Blackwell Science p5 (2001)
Non-Patent Document 3: Redgwell R J., Fischer M., Curti D., Sutherland P., Haallett I., Macrae E., Arabinogalactan-proteins in Coffee Bean Cell Walls, FFI Journal, 211, 38-47 (2006)
Non-Patent Document 4: Lisa Imamura, Kenji Murai, Chun-Ju Zhao, Sachiko Takebe, Kyoichi Kobashi, Metabolism of Arabinogalactan by Rat and Human Enterobacterium, Bifidus, 6, 19-29 (1992)
Non-Patent Document 5: R.J. Clarke, O.G. Vitzhum, COFFEE Recent Developments, Blackwell Science p4 (2001)

### SUMMARY OF THE INVENTION

### PROBLEMS TO BE SOLVED BY THE INVENTION

The object of the present invention is to provide a method of efficiently producing polysaccharides from coffee beans or/and a coffee extraction residue which have not easily been decomposed and have been done in the past focused on heretofore, especially to provide a method of efficiently isolating arabinogalactan or galactan and galactomannan.

As a result of the extensive study to solve the above problems, it has been found that the above object can be achieved by the following method of producing polysaccharides from coffee beans or/and a coffee extraction residue, and the present invention was thereby completed.

That is, the present invention relates to a method of producing polysaccharides from coffee beans or/and a coffee extraction residue, comprising:
(a) step of milling coffee beans or/and a coffee extraction residue to the particle which diameter is from 10.0 µm to 5.0 mm,
(b1) step of conducting heat treatment at 50 to 100°C in the presence of diluted alkali, after the step of (a),
(c1) step of treating with cellulase, after the step of (b1),
(b2) step of conducting heat treatment at 120°C or more in the presence of diluted alkali, after the step of (c1), and
(c2) step of treating with cellulase, after the step of (b2) (claim 1).

Namely, according to the present invention, it has been found that, by repeating a method of subjecting coffee beans or a coffee extraction residue to an alkali treatment and then treating with an enzyme derived from microorganism, particularly cellulase, a cell wall constituent component of coffee beans is directly treated thereby isolating monosaccharides and oligosaccharides derived from cellulose, and thus arabinogalactan and galactomannan containing a large amount of galactose can be effectively extracted. As a result, it became possible to provide a method of efficiently producing polysaccharides from coffee beans or a coffee extraction residue which have not easily been decomposed heretofore.

In the present invention, 85% or more of the cell wall component of coffee beans and/or a coffee extraction residue is solubilized (claim 2).

As described above, arabinogalactan is a cell wall constituent component and it was very difficult to extract from coffee beans and/or a coffee extraction residue by a conventional method. According to the present invention, it has been found that 85% or more of the cell wall component can be solubilized by optimizely using two-stage alkali and enzyme treatments in combination under predetermined temperature conditions. Thus, it became possible to efficiently extract polysaccharides such as arabinogalactan and galactomannan.

In the present invention, the cell wall component is composed of arabinogalactan and galactan to form a cell wall structure (claim 3).

As a result of the above verification, it has been found that not only arabinogalactan or galactan but also a galactomannan portion having a large amount of galactose, which has commercially high value, exists in the cell wall of coffee beans. In the present invention, it became possible to extract arabinogalactan and galactomannan, efficiently and mildly, in a simple and easy manner from such a cell wall component by subjecting coffee beans or a coffee extraction residue to a predetermined treatment.

In the present invention, the polysaccharides include arabinogalactan or galactan (claim 4).

As described above, coffee beans contain arabinogalactan and it is very difficult to extract arabinogalactan. It is necessary to extract with an alkali having very high concentration for a long time so as to extract arabinogalactan by a conventional method. In the present invention, it became possible to efficiently extract arabinogalactan or galactan in a simple and easy manner from such a cell wall component by subjecting coffee beans or a coffee extraction residue to a predetermined treatment.

In the present invention, the polysaccharides include galactomannan containing 30% or more of galactose (claim 5).

As a result of the above verification, it has been found that only conventional galactomannan having a very small ratio of galactose to mannose is not distributed on the cell wall of coffee beans and a galactomannan portion having a large ratio of galactose to mannose (30% or larger), which has commercially high value, exists. In the present invention, it became possible to extract arabinogalactan and galactomannan, efficiently and mildly, in a simple and easy manner by the method described above.

### EFFECT OF THE INVENTION

According to the present invention, arabinogalactan having arabinose and galactan in a ratio of 1:2.4 to 1:4, and galactan composed of galactose and galactomannan having galactose and mannose in a ratio of 1:1 to 1:3 can be effectively obtained by using coffee beans or a coffee extraction residue as raw materials. Galactomannan and arabinogalactan, which contain a large amount of galactose, can be used as thickening polysaccharides in the fields of food industry, cosmetics and medical supplies, and can be efficiently produced using coffee beans or a coffee extraction residue as raw materials.

### BRIEF DESCRIPTION OF THE DRAWINGS

Fig. 1 is a flow diagram showing a method of producing polysaccharides from coffee beans or a coffee extraction residue.
Fig. 2 is an explanatory graph showing a relation between the results of an alkali treatment and the treatment time.
Fig. 3 is an explanatory graph showing a relation between the results of an alkali treatment and the treatment temperature.
Fig. 4 is an explanatory graph showing a relation between the results of an alkali treatment and the treatment temperature.
Fig. 5 is an explanatory view showing the results of an enzyme treatment after an alkali treatment.
Fig. 6 is an explanatory graph showing a relation between the results of an enzyme treatment and the treatment time.
Fig. 7 is an explanatory graph showing the constitution of a saccharide produced during an enzyme treatment.
Fig. 8 is an explanatory view showing a structure of a cell wall after the enzyme treatment.
Fig. 9 is an explanatory view showing a treating step according to Example 1 and a saccharide composition of an extract solution and a solid residue potion.
Fig. 10 is an explanatory view showing a treating step according to Example 2 and a saccharide composition of an extract solution and a solid residue potion.
Fig. 11 is an explanatory view showing a treating step according to Example 3 and a saccharide composition of an extract solution and a solid residue potion.

### BEST MODE FOR CARRYING OUT THE INVENTION

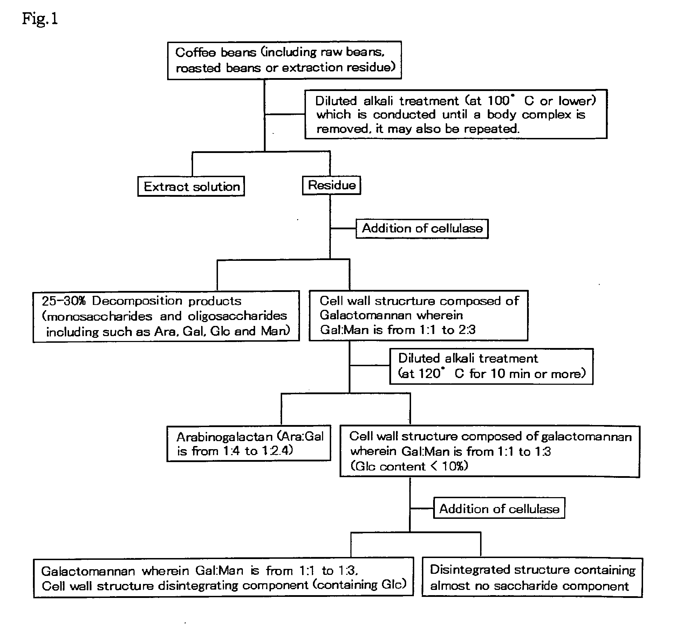

The present invention will be described in detail by a method of producing arabinogalactan and galactomannan derived from coffee beans or a coffee extraction residue with reference to a flow chart shown in Fig. 1. An alkali treatment and an enzyme treatment are not limited thereto and good results are obtained by appropriately repeating these treatments. However, yield of galactomannan increases when the alkali treatment and the enzyme treatment are alternately repeated.

Coffee beans used in the present invention are derived from three foundation seeds of cultivated species of the genus Coffea belonging to madder plants, for example, Coffea arabica, Coffea canephora and Coffea liberica, and several tens kinds of coffee plants derived therefrom. Coffea Arabica occupies about two-thirds of the production amount of coffee in the world and has a feature such as high quality and rich aroma. Coffea canephora has strong bitterness and also has no sourness and does not have rich aroma, and is therefore suited for blending. Coffea liberica has low quality and is produced in a small amount and is not imported into Japan.

The present invention relates to a method of producing polysaccharides from coffee beans or/and a coffee extraction residue, comprising:
(a) step of milling coffee beans or/and a coffee extraction residue,
(b1) step of conducting heat treatment in the presence of diluted alkali (the first alkali treating step is the step 'b1'),
(c1) step of treating with enzyme cellulase (the first enzyme treating step is the step 'c1'),
(b2) step of another conducting heat treatment in the presence of diluted alkali (the second alkali treating step is the step 'b2'), and
(c2) step of another treating with enzyme cellulase (the second enzyme treating step is the step 'c2') (claim 1).
   Namely, a cell wall constituent component of coffee beans is directly treated thereby isolating monosaccharides and oligosaccharides derived from cellulose, and thus arabinogalactan and galactomannan containing a large amount of galactose can be effectively extracted. Description will be made below by way of each step.

### (a) Step of Milling Coffee Beans or Coffee Extraction Residue

In the present invention, it is possible to use coffee beans obtained with any derivation or by any process. However, it is preferred to increase surface area by milling coffee beans into powders using various mills so as to efficiently produce arabinogalactan and galactomannan. Namely, when a coffee extraction residue discharged from a coffee beverage factory is used as it is, even if the objective product can be obtained, the yield is low. So by milling the coffee extraction residue, the disintegration rate of coffee beans increases by 2 to 3 times and the yield of arabinogalactan and galactomannan increases. Specifically, the coffee extraction residue can be milled using dry mills such as jet-, tornado-, ball- and cutter-type mills. When the coffee extraction residue is finely milled, the enzyme reaction proceeds more easily.

Regarding the particle size, the particle diameter (median value) is preferably from 10.0 µm to 5.0 mm, and more preferably from 10.0 µm to 0.6 mm. When the particle diameter is less than 10.0 µm, it becomes difficult to convey or handle because fine powders are formed. In contrast, when the particle diameter is 5.0 mm or more, a treating function is not sufficiently exerted and an extraction efficiency decreases.

When the coffee beans or coffee extraction residue contain a large amount of fat, they may be appropriately defatted with such as hot water, steam or an organic solvent. Also, an organic solvent or a mixed solvent of an organic solvent and water can be used. Specific examples of the organic solvent include such as ethanol, methanol, propanol, ethylether, hexane and chloroform.

### (b1) Step of Conducting Heat Treatment in the Presence of Diluted Alkali (First Time)

In the present invention, a pretreatment with an alkali is conducted as the pretreatment process. It has been found that by conducting an alkali treatment as the pretreatment process, a body complex, sucrose, polyphenol, water soluble polysaccharides and the like existing in the structure of coffee beans or a brown pigment and water soluble polysaccharides existing in the coffee extraction residue are removed through extraction, and the subsequent enzyme reaction easily proceeds.

The body complex means a portion which is included in cells of the albumen portion of plants, and is in the form of a lump in which lipid , protein and the like inside of a primary cell wall are coated with a film made of pectin and the like. In the case of soybeans, a typical body structure can be observed (Kasai N et al., Isolation and Enzymatic Digestion of Body Complex of Soybean Seed, J. Agric Food Chem, 53, 10026-10033 (2005)).

It is possible to use, as the aqueous alkali solution, those approximately selected from aqueous solutions of sodium carbonate, sodium hydroxide, potassium hydroxide, barium hydroxide and the like. The amount of the aqueous alkali solution is preferably a 5- to 30-fold amount of dry coffee raw beans, roasted coffee beans or a coffee extraction residue.

When coffee raw beans or roasted coffee beans are used, a similar pretreatment is required. In the roasted coffee beans or coffee extraction residue, since a coffee brown pigment as a thermal reaction product of such as sucrose, protein or polyphenol adheres to the cell wall component, this alkali treatment may be repeated twice or more.

### (b1-1) Study of Alkali Treatment Concentration and Treatment Time

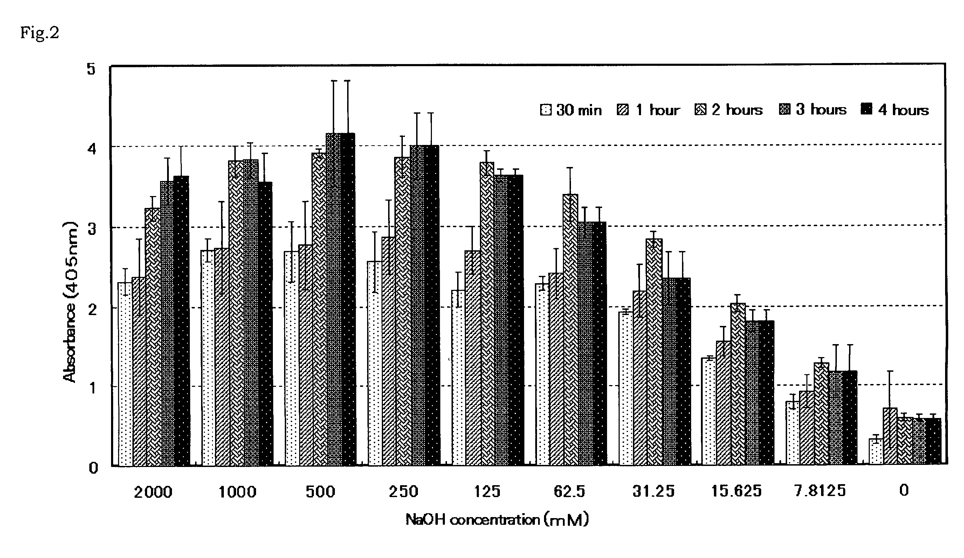

Since the alkali treatment concentration and the time are preferably set to conditions as mild as possible, the alkali concentration and the time were studied.
(1) In the study, 0.1 g of a coffee extraction residue was specifically used and, after treating with a 50-fold amount of the above NaOH solution, the eluted brown substance was measured at an absorbance of 405 nm. A NaOH solution having an alkali concentration of 2,000 mM (mol%), 1,000 mM, 500 mM, 250 mM, 125 mM, 62.5 mM, 31.25 mM, 15.63 mM, 7.813 mM or 0 mM (distilled water, control) was used. The study was made under the conditions of the treating time of 30 minutes, 1 hour, 2 hours, 3 hours and 4 hours and the treatment temperature of 120°C.
(2) All tests were conducted four times, and the resultant averages and SD (standard deviation) were shown in graphs. As a result, as shown in Fig. 2, in the alkali pretreatment, the amount of the brown substance to be eluted tends to increase as the treatment time increases at any alkali concentration. However, in the test section at the alkali concentration within a range from 125 to 2,000 mM, a significant difference was not recognized when analysis of variance and multiple comparison were conducted in the amount of the brown pigment eluted by the treatment for 4 hours, for example. In the test section at the alkali concentration within a range from 0 to 125 mM, a significant difference at a significance level of 5% was entirely recognized when analysis of variance and multiple comparison were conducted in the amount of the brown pigment eluted by the treatment for 4 hours, for example.

### (b1-2) Study of Alkali Treatment Concentration and Treatment Temperature

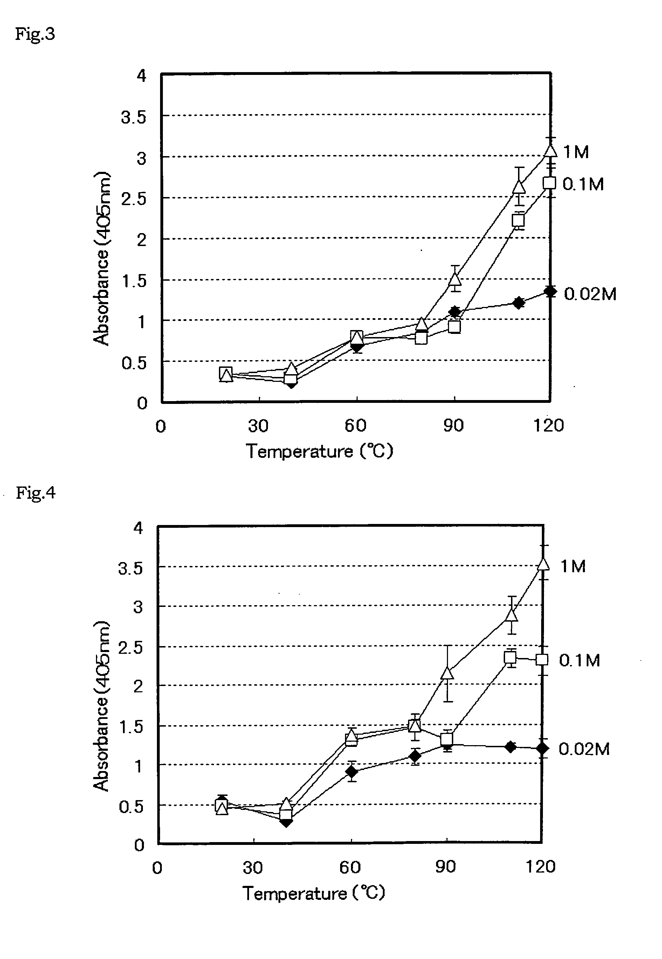

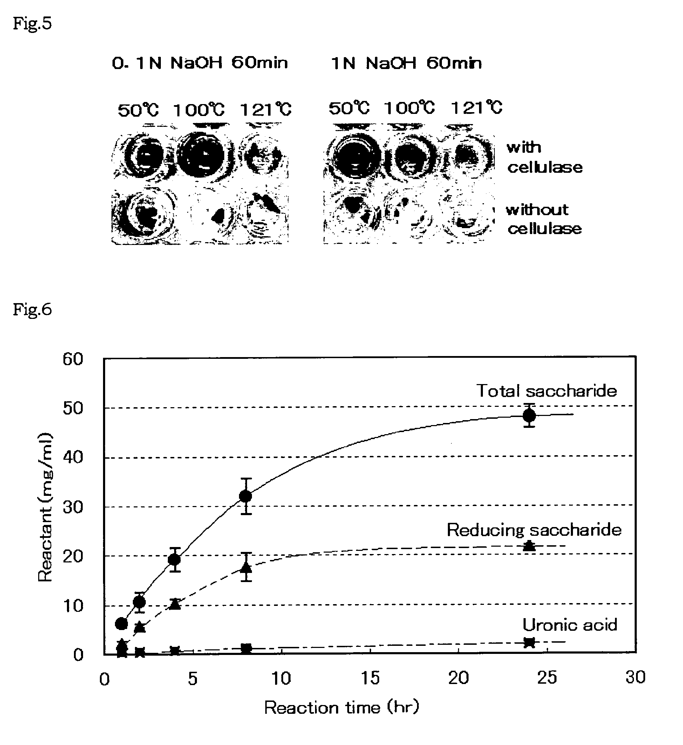

A relation between the alkali treatment concentration and the treatment temperature was studied.
(1) In the study, a NaOH solution having an alkali concentration of 0.02 M, 0.1 M or 1 M was used in the alkali pretreatment. The study was made under the conditions of the treating temperature of 20°C, 40°C, 60°C, 80°C, 90°C, 110°C and 120°C and the treatment time of 1 hour and 2 hours. After subjecting to the pretreatment under the above conditions, an enzyme treatment was conducted using cellulase as an enzyme derived from microorganism and a presence or an absence of a coffee extraction residue exists was determined.
(2) As the results of the case where the pretreatment was conducted, as shown in Fig. 3 (treatment time: 1 hour) and Fig. 4 (treatment time: 2 hours), regarding the treatment concentration, the amount of the brown pigment to be eluted tends to increase depending on the concentration. However, there was not a large difference in the amount of the brown pigment to be eluted between 1 M NaOH and 0.1 M NaOH. Regarding the treatment temperature, the amount of the brown pigment to be eluted increased at the temperature of 90°C or higher in both cases of the treatment time of 1 hour and 2 hours.
   The results of the case where the enzyme treatment was conducted are shown in a micrograph of Fig. 5. When a pretreatment was conducted at 100°C or lower for 1 hour using 0.1 M NaOH or a pretreatment was conducted at 50°C or lower for 1 hour using 1 M NaOH, the existence of a coffee extraction residue was confirmed. Then in the test section of the subsequent enzyme treatment, when 0.1 M NaOH was used in and a pretreatment was conducted at 50°C for 1 hour, disintegration of the coffee extraction residue was recognized, and when 0.1 M NaOH was used and a pretreatment was conducted at 100°C for 1 hour, the coffee extraction residue was disintegrated.

### (bl-3) Summary of the Study Results

From the above study results, the concentration of the aqueous alkali solution in the alkali treatment is preferably 0.1 M or more, and more preferably from 0.1 to 1 M. The alkali treatment time is preferably about 1 hour or more, and more preferably a pretreatment is conducted under a heating condition for 2 or more hours.
The alkali treatment temperature is preferably a heating temperature of 90°C or higher so as to increase the amount of the brown substance to be eluted. It is preferably a heating temperature of 50°C or higher so as to cause disintegration of the coffee extraction residue in the enzyme treatment in the post-process. Furthermore, it is preferable to conduct a pretreatment under a heating temperature of 90°C or higher so as to make a state free from the coffee extraction residue in appearance. Provided that it is possible to make a state where the coffee extraction residue scarcely exists by adjusting the concentration of the aqueous alkali solution even under the heating temperature of 90°C or lower. The pretreatment temperature is preferably low temperature so as to early realize the conditions where the enzyme used in the enzyme treatment is not deactivated (for example, 60°C or lower). When a two-stage heat treatment (b2) and a two-stage enzyme treatment (c2) described hereinafter are conducted, a first alkali treatment is conducted for the purpose of removing the brown pigment and the body complex, and the heat treatment at an early stage is preferably conducted at a temperature of 50 to 100°C.
When the amount of the alkali is increased and the treatment temperature is raised, the number of the alkali treatment can also be decreased. In this case, the amount of the alkali is preferably from a 5-to 30-fold amount, and particularly preferably a 10-fold amount or more. The conditions are preferably as follows: the concentration of the alkali is 0.1 M or more, the treatment temperature is 100°C or higher, and the treatment time is 20 minutes or more.

### (c1) Step of Treating with Enzyme Cellulase (First Time)

The conditions of the enzyme reaction of the alkali-treated coffee beans and coffee extraction residue will be described below. The enzyme used in the present invention is derived from microorganism and is not specifically limited as long as it has an activity capable of disintegrating the cell wall component by treating with the coffee beans and coffee extraction residue. Microorganism are bacteria which have been used for a long time as bacteria capable of producing an enzyme such as pectinase used to clarify juice and hemicellulase used to improve an extraction rate of coffee, and also have established safety.

Such an enzyme can be obtained by culturing microorganism. The culturing process is not specifically limited as long as it is a process capable of producing a polysaccharides disintegrating enzyme, and a conventional culturing process such as solid or liquid culturing process may be used. Culturing may be conducted under conventional culturing conditions. In the present invention, any fraction having an activity capable of disintegrating coffee beans and a coffee extraction residue. If necessary, it is also possible to use those obtained by purification or partial purification of the fraction having an activity capable of disintegrating the coffee beans and coffee extraction residue. Also, a commercially available exogenous enzyme may be used and examples of the exogenous enzyme include such as cellulase DAIWA (Daiwa Fine Chemicals Co., Ltd.). This exogenous enzyme is commercially available as an exogenous enzyme for food, and has already been used in foods and also has established safety. Cellulase DAIWA is a kind of cellulases produced by microorganism of the genus Trichoderma.

The conditions for treatment of the coffee beans and coffee extraction residue with the above enzyme may be any conditions used in a conventional enzyme reaction. However, the reaction preferably arises under optimum operating conditions of the enzyme used. The reaction is preferably conducted under the conditions where the enzyme is not deactivated and the reaction temperature is preferably from 25 to 65°C, and more preferably from 50 to 60°C. About pH of the reaction, the reaction is preferably conducted under the optimum operating conditions of the enzyme as a mater of course, and pH is preferably from 2 to 9, and more preferably from 4.0 to 6.0.

### (c1) Study on Enzyme Reaction

A coffee extraction residue was disintegrated using an enzyme derived from microorganism and the produced saccharides were analyzed and what was produced was studied.
(1) To 2.0 g of an alkali-treated coffee extraction residue (which is obtained by adding a 50-fold amount of 0.1 M NaOH to a coffee extraction residue, followed by autoclaving at 120°C for 1 hour, filtration and further drying), 15 mL of a 0.1 M acetate buffer (pH 5.0) and 1 mL of an enzyme solution prepared by dissolving 80 mg of cellulase DAIWA (Daiwa Fine Chemicals Co., Ltd.) in 1 mL of a 0.1 M acetate buffer (pH 5.0) were added and an enzymatic hydrolysis reaction was conducted at 40°C. 1, 2, 4, 8 and 24 hours after the initiation of the reaction, sampling was conducted. After centrifugal separation, an enzyme reaction was terminated by heating to a deactivation temperature (70°C) of the enzyme. With respect to each sample, a neutral saccharide was analyzed by an alditol acetate process.
(2) As shown in Fig. 6, a reducing saccharide and uronic acid are similarly produced as the increase of the total amount of the saccharide increases with the proceeding of the enzyme reaction. As the results of the graph, the amount of saccharides to be produced reaches to the upper limit after 24 hours of the enzyme reaction, and thus it is considered that the enzyme treatment time is preferably about 24 hours. From the amount of the total saccharide (48.05 mg/mL) and the amount of the reducing saccharide (21.77 mg/mL) after 24 hours of the enzyme reaction, it becomes 48.05/21.77 ≈ 2.21. Therefore, it is considered that an average saccharide chain number of the saccharide to be produced by the enzyme reaction is about 2.21. From the above results, the enzyme reaction time, which depends on the amount of the enzyme to be used, is preferably set within a range from 8 to 24 hours from an operational point of view.

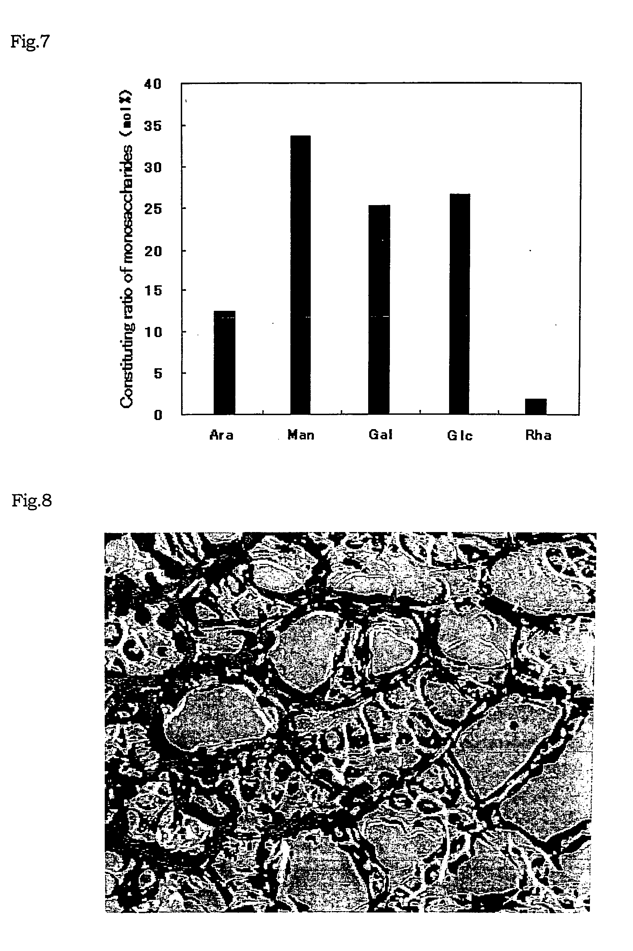

(c1-2) Analysis of Constitution of Saccharide Produced
(1) Kinds of the reducing saccharide contained in a solution were analyzed. As a result, arabinose (Ara), galactose (Gal), mannose (Man), glucose (Glc) and rhamnose (Rha) were detected as monosaccharides as shown in Fig. 7. These saccharides are not originally contained in the coffee extraction residue, thus it is considered that these saccharides are derived from polysaccharides constituting the coffee extraction residue, namely, arabinogalactan and galactomannose containing a large amount of mannose, and are derived from constitution components of cellulose. Thus, it is considered that these monosaccharides are produced by enzymatic hydrolysis of coffee beans and the cell wall component of the coffee extraction residue through an enzyme produced by microorganism.
(2) With respect to the portion which was not disintegrated by the above enzyme treatment, the saccharide was analyzed. As a result, it has been found that it is a cell wall component composed of polysaccharides containing galactose and mannose in a ratio of 1:1 to 2:3.
(3) It has also been found that the polysaccharides component containing galactose and mannose in a ratio of 1:1 to 2:3 is partially contained in the cell wall of coffee beans and is a main constituent component of a structure included inside the cell in the form of a network (Fig. 8). This polysaccharides component could be easily recovered by a centrifugation treatment at 3,000 rpm for 5 minutes.

### (b2) Step of Conducting Heat Treatment in the Presence of Diluted Alkali (Second Time)

It is possible to separately isolate arabinogalactan having a ratio of arabinose to galactose of 1:4 to 1:2.4 and galactomannan constituting protein having a ratio of galactose to mannose of 1:1 to 1:3 by additionally treating the polysaccharides component derived from cell wall obtained by the above method with 0.1 M NaOH at 120°C for 10 minutes.

At this time, regarding the conditions of the alkali treatment, as described in (b1-3), the concentration of an aqueous alkali solution is preferably 0.1 M or more, and more preferably from 0.1 to 1 M. The alkali treatment time is preferably about 1 or more hours, and more preferably a pretreatment is conducted under heating conditions for 2 or more hours. It is preferred to conduct a pretreatment under a heating condition at a temperature of 120°C or higher so as to increase a brown substance to be eluted and to make a state free from a coffee extraction residue in an enzyme treatment at a post-process.

Namely, a first alkali treatment is conducted for the purpose of removing a brown pigment and a body complex and may be conducted at a temperature of 50 to 100°C. However, a second alkali treatment must be at a temperature of 120°C or higher. As a result, arabinogalactan bonded firmly to a cell wall is eluted at the same temperature and a matrix part is properly exposed, thus allowing the subsequent enzyme reaction to proceed.

### (c2) Step of Treating with Enzyme Cellulose (Second Time)

Galactomannan with further enhanced purity can be obtained by treating the resultant galactomannan constituting portion having galactose and mannose in a ratio of 1:1 to 1:3 with cellulose again.

As described above, in the present invention, at least 85% or more of the cell wall component of coffee beans and a coffee extraction residue is solubilized by the production method in which two-stage alkali and enzyme treatments are optimizely used in combination. An enzyme treating solution contains monosaccharide, oligosaccharide, arabinogalactan and galactomannan containing the content of galactose of 30% or more, but contains no cellulose. When only arabinogalactan or galactomannan is isolated, purification can be conducted by a method of adding ethanol and recovering the resultant precipitate through a treatment such as centrifugal separation, or chromatography such as gel filtration. Alternately, a solubilized solution may be appropriately concentrated and dried in the form of a mixture.

### EXAMPLES

The present invention will be described in detail below by way of examples.

### Example 1: Production of Arabinogalactan (Galactan) and Galactomannan from Coffee Extraction Residue

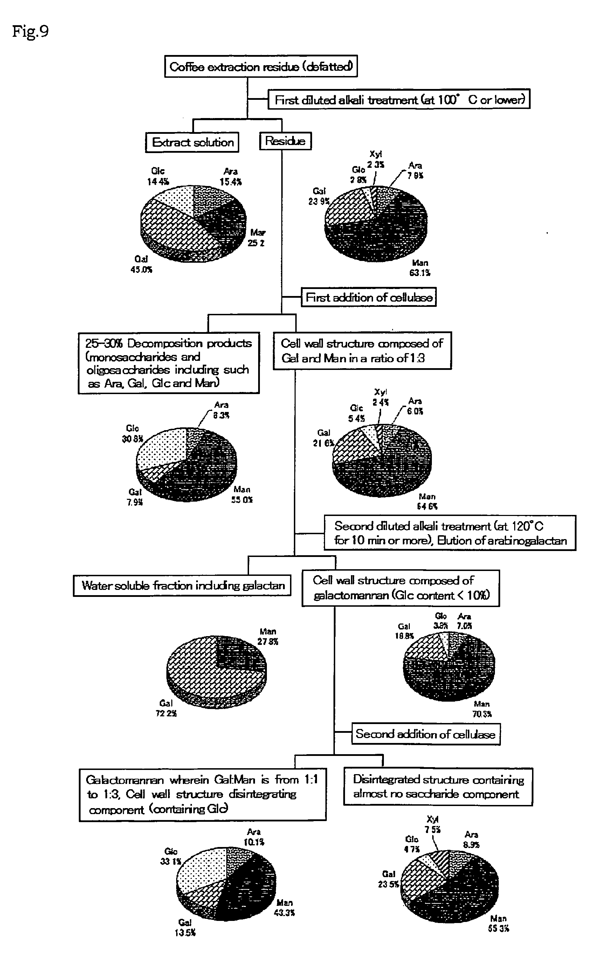

A series of treatment steps and the saccharide composition of the extract solution and the solid residue portion are shown in Fig. 9.

### (1) Diluted Alkali Treatment (First Time)

To 2 g of a defatted coffee extraction residue, a 10-fold amount of a 0.1 M sodium carbonate buffer (pH 10.0) was added, followed by heating at 100°C for 20 minutes. After the heat treatment, solid-liquid separation was conducted by centrifugal separation (at 3,000 rpm for 5 minutes) to obtain an alkali-treated coffee extraction residue. To 2 g of the alkali-treated coffee extraction residue obtained by this treatment, a 10-fold amount of 0.1 M sodium hydroxide was added, followed by heating at 100°C for 20 minutes. After the heat treatment, solid-liquid separation was conducted by centrifugal separation (at 3,000 rpm for 5 minutes). The saccraride content in the supernatant was 425.3 mg. The precipitated residue was recovered.

### (2) Enzyme Treatment (First Time)

To 0.91 g of the alkali-treated coffee extraction residue obtained by the treatment (1), a 10-fold amount of a 0.1 M acetate buffer (pH 5.0) was added and cellulase DAIWA (Daiwa Fine Chemicals Co., Ltd.) was added in the concentration of 1%, and then the reaction was conducted at 40°C for 24 hours. After the reaction, solid-liquid separation was conducted by centrifugal separation (at 3,000 rpm for 5 minutes). The saccharide content of the supernatant was 955.0±111 mg. The precipitated residue was a cell wall component containing galactomannan as a main component, and the amount of the cell wall component was 20.5% by weight of an initial untreated coffee extraction residue. A ratio of galactose to mannose was 1:3.

### (3) Diluted Alkali Treatment (Second Time)

To 0.44 g of the cell wall component obtained by the treatment (2), a 10-fold amount of 0.1 M sodium hydroxide was added, followed by heating at 120°C for 20 minutes. After the heat treatment, an extract solution containing galactose and mannose in a ratio of 1:2.6 was obtained. Galactose in the extract solution is contained as a polymer and is found to be derived from arabinogalactan. Solid-liquid separation of the treating solution was conducted by centrifugal separation (at 3,000 rpm for 5 minutes). The sacharide content of the supernatant was 59.5±3.7 mg. The precipitated residue was a cell wall component having further enhanced content of galactomannan and the amount of this component was 13.5% by weight of an initial untreated coffee extraction residue.

### (4) Enzyme Treatment (Second Time)

0.21 g of the cell wall component obtained by the treatment (3) was dissolved in a 10-fold amount of 0.1 M acetate buffer (pH 5.0) and cellulase DAIWA (Daiwa Fine Chemicals Co., Ltd.) was added in the concentration of 1%, and then the reaction was conducted at 40°C for 24 hours. After the completion of the reaction, solid-liquid separation was conducted by centrifugal separation (at 3,000 rpm for 5 minutes) to obtain a solubilized solution containing galactomannan. The saccraride content of the supernatant was 177.9±24 mg and a ratio of galactose to mannose was 1:3.

### (5) Results

As shown in Table 1, the defatted coffee extraction residue finally contained 3.85% of the insoluble component as a result of a series of treatments. The final residue component mainly contained mannose. However, the insoluble component contained a very small amount of saccharide and was easily disintegrated when a pressure is applied using a finger.

**Table 1**

| Treatment method (2 g) | Water soluble fraction | | | Residual ratio (%)* |
|---|---|---|---|---|
| | Total saccharide (mg) | Uronic acid (mg) | Protein (mg) | Average ± standard deviation |
| (1) Diluted alkali treatment of coffee extraction residue (0.1 M Na₂CO₃ buffer - 0.1 M NaOH treatment, each at 100°C for 20 min) | 425.31 ± 29.56 | 26.75 ± 2.30 | 276 ± 16.47 | 45.56 ± 2.78 |
| (2) Cellulase treatment to residue of (1) (First time) | 955.04 ± 110.98 | 22.94 ± 1.92 | 8.28 ± 1.80 | 22.15 ± 0.35 |
| (3) Diluted alkali treatment to residue of (2) (0.1 M NaOH treatment at 120°C for 20 min) | 59.48 ± 3.67 | 2.66 ± 0.40 | 29.82 ± 1.99 | 10.5 ± 0.33 |
| (4) Cellulase treatment to residue of (3) (Second time) | 177.86 ± 24.05 | 4.31 ± 0.70 | 11.69 ± 2.71 | 3.85 ± 0.06 |

| | | | | |
|---|---|---|---|---|
| Here, Residual ratio (%)* = [(Weight of water insoluble solid) / (Weight of untreated beans)] × 100 (n = 4) | | | | |

### Example 2: Production of Arabinogalactan and Galactomannan from Raw Coffee Beans

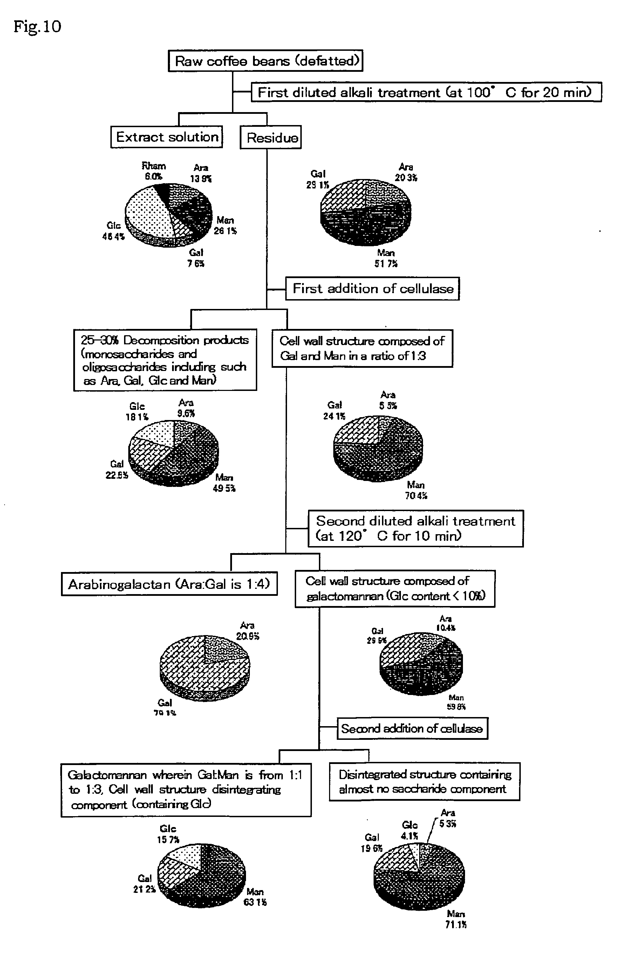

A series of treatment steps and the saccharide composition of the extract solution and the solid residue portion are shown in Fig. 10.

### (1) Diluted Alkali Treatment (First Time)

Raw coffee beans (Brazil Santos No. 2) were milled (average particle diameter: 78.7 µm, median diameter: 45.4 µm) and defatted and a 10-fold amount of a 0.1 M sodium carbonate buffer (pH 10.0) was added to 2.0 g of the resultant coffee beans, followed by heating at 100°C for 20 minutes. After the heat treatment, solid-liquid separation was conducted by centrifugal separation (at 3,000 rpm for 5 minutes) to obtain alkali-treated raw coffee beans. To 2 g of the alkali-treated raw coffee beans obtained by this treatment, a 10-fold amount of 0.1 M sodium hydroxide was added, followed by heating at 100°C for 20 minutes. After the heat treatment, the residue precipitated by centrifugal separation (at 3,000 rpm for 5 minutes) was recovered. The saccraride content in the supernatant was 421.0 mg. The precipitated residue was recovered.

### (2) Enzyme Treatment (First Time)

To 0.93 g of the alkali-treated coffee beans obtained by the treatment (1), a 10-fold amount of a 0.1 M acetate buffer (pH 5.0) was added and cellulase DAIWA (Daiwa Fine Chemicals Co., Ltd.) was added in the concentration of 1%, and then the reaction was conducted at 40°C for 24 hours. After the reaction, solid-liquid separation was conducted by centrifugal separation (at 3,000 rpm for 5 minutes). The saccharide content of the supernatant was 940.9±77 mg. The precipitated residue was a cell wall component containing arabinogalactan and galactomannan as a main component and a ratio of galactose to mannose of the cell wall component was 1:3. The amount of the cell wall component was 22.2% by weight of initial untreated raw coffee beans.

### (3) Diluted Alkali Treatment (Second Time)

To 0.41 g of the cell wall component obtained by the treatment (2), a 10-fold amount of 0.1 M sodium hydroxide was added, followed by heating at 120°C for 20 minutes. After the heat treatment, a water soluble fraction containing arabinogalactan bonded firmly to the cell wall component was eluted. A ratio of arabinose to galactose of the fraction was 1:4. Solid-liquid separation of the treating solution was conducted by centrifugal separation (at 3,000 rpm for 5 minutes). The saccharide content of the supernatant was 31.7±7.9 mg. The precipitated residue was a cell wall component having further enhanced content of galactomannan and the amount of this component was 13.5% by weight of an initial untreated coffee extraction residue.

### (4) Enzyme Treatment (Second Time)

0.27 g of the cell wall component obtained by the treatment (3) was dissolved in a 10-fold amount of 0.1 M acetate buffer (pH 5.0) and cellulase DAIWA (Daiwa Fine Chemicals Co., Ltd.) was added in the concentration of 1%, and then the reaction was conducted at 40°C for 24 hours. After the reaction, solid-liquid separation was conducted by centrifugal separation (at 3,000 rpm for 5 minutes) to obtain a solubilized solution containing galactomannan. The saccharide content of the supernatant was 394.2±28.3 mg and a ratio of galactose to mannose was 1:3.

### (5) Results

As shown in Table 2, the defatted raw coffee beans finally contained 4.95% of the insoluble component as a result of a series of treatments. The final residue component mainly contained mannose. However, the insoluble component contained a very small amount of saccharide and was easily disintegrated when a pressure is applied using a finger.

**Table 2**

| Treatment method (2 g each) | Water soluble fraction | | | Residual ratio (%)* |
|---|---|---|---|---|
| | Total saccharide (mg) | Uronic acid (mg) | Protein (mg) | Average ± standard deviation |
| (1) Diluted alkali treatment of raw coffee beans (0.1 M Na₂CO₃ buffer - 0.1 M NaOH treatment, each at 100°C for 20 min) | 420.96 ± 50.16 | 30.28 ± 3.35 | 204.37 ± 24.89 | 46.34 ± 1.24 |
| (2) Cellulase treatment to residue of (1) (First time) | 940.86 ± 77.05 | 33.07 ± 2.13 | 4.97 ± 1.27 | 20.5 ± 0.21 |
| (3) Diluted alkali treatment to residue of (2) (0.1 M NaOH treatment at 120°C for 20 min) | 31.66 ± 7.91 | 3.66 ± 0.29 | 12.72 ± 1.68 | 13.5 ± 0.20 |
| (4) Cellulase treatment to residue of (3) (Second time) | 394.19 ± 28.28 | 11.02 ± 1.07 | 13.56 ± 1.26 | 4.95 ± 0.12 |

| | | | | |
|---|---|---|---|---|
| Here, Residual ratio (%)* = [(Weight of water insoluble solid) / (Weight of untreated beans)] × 100 (n = 4) | | | | |

### Example 3: Production of Arabinogalactan and Galactomannan from Raw Coffee Beans

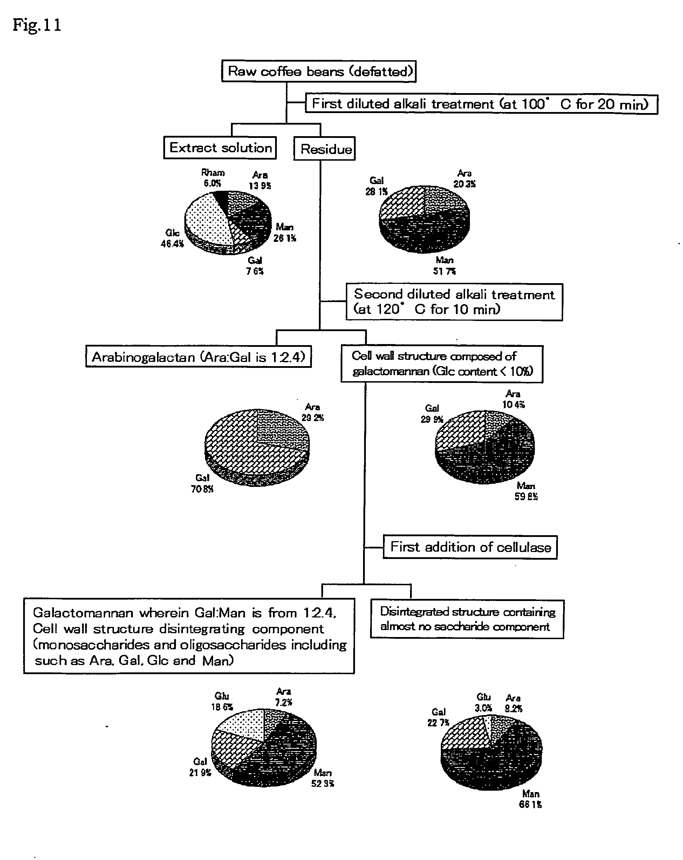

A series of treatment steps and the saccharide composition of the extraction solution and the solid residue portion are shown in Fig. 11.

### (1) Diluted Alkali Treatment (First Time)

Raw coffee beans (Brazil Santos No. 2) were milled (average particle diameter: 78.7 µm, median diameter: 45.4 µm) and defatted and a 10-fold amount of a 0.1 M sodium carbonate buffer (pH 10.0) was added to 2.0 g of the resultant coffee beans, followed by heating at 100°C for 20 minutes. After the heat treatment, solid-liquid separation was conducted by centrifugal separation (at 3,000 rpm for 5 minutes) to obtain alkali-treated raw coffee beans. To 2 g of the alkali-treated raw coffee beans obtained by this treatment, a 10-fold amount of 0.1 M sodium hydroxide was added, followed by heating at 100°C for 20 minutes. After the heat treatment, solid-liquid separation was conducted by centrifugal separation (at 3,000 rpm for 5 minutes). The saccraride content in the supernatant was 421.0±50.2 mg. The precipitated residue was recovered.

### (2) Diluted Alkali Treatment (Second Time)

To 0.93 g of the alkali-treated raw coffee beans obtained by the treatment (1), a 10-fold amount of 0.1 M sodium hydroxide was added, followed by heating at 120°C for 20 minutes. After the heat treatment, solid-liquid separation of this treating solution was conducted by centrifugal separation (at 3,000 rpm for 5 minutes) to obtain a water soluble fraction containing arabinogalactan and a cell wall component containing galactomannan. A ratio of arabinose to galactose of the water soluble fraction containing arabinogalactan was 1:2.4 and the saccharide content in the water soluble fraction was 94.9±10.3 mg. The amount of the cell wall component containing galactomannan was 40.5% by weight of initial untreated raw coffee beans.

### (3) Enzyme Treatment (First Time)

0.8 g of the cell wall component containing galactomannan obtained by the treatment (2) was dissolved in a 10-fold amount of a 0.1 M acetate buffer (pH 5.0) and cellulase DAIWA (Daiwa Fine Chemicals Co., Ltd.) was added in the concentration of 1%, and then the reaction was conducted at 40°C for 24 hours. After the reaction, solid-liquid separation was conducted by centrifugal separation (at 3,000 rpm for 5 minutes) to obtain a solubilized solution containing 903±27.2 mg of monosaccharide, oligosaccharide and galactomannan. A ratio of galactose to mannose of galactomannan was 1:2.4.

### (4) Results

As shown in Table 3, the defatted raw coffee beans finally contained 13.7% of the insoluble component as a result of a series of treatments. The final residue component mainly contained mannose. However, the insoluble component contained a very small amount of saccharide and was easily disintegrated when a pressure is applied by a finger.

**Table 3**

| Treatment method (2 g each) | Water soluble fraction | | | Residual ratio (%)* |
|---|---|---|---|---|
| | Total saccharide (mg) | Uronic acid (mg) | Protein (mg) | Average ± standard deviation |
| (1) Diluted alkali treatment of raw coffee beans (0.1 M Na₂CO₃ buffer - 0.1 M NaOH treatment, each at 100°C for 20 min) | 420.96 ± 50.16 | 30.28 ± 3.35 | 204.37 ± 24.89 | 46.34 ± 1.24 |
| (2) Cellulase treatment to residue of (1) (First time) | None | | | |
| (3) Diluted alkali treatment to residue of (2) (0.1 M NaOH treatment at 120°C for 20 min) | 94.86 ± 10.33 | 10.58 ± 0.61 | 14.68 ± 1.50 | 40.05 ± 1.75 |
| (4) Cellulase treatment to residue of (3) (Second time) | 902.09 ± 27.19 | 23.88 ± 1.89 | 0 ± 0.00 | 13.7 ± 0.01 |

| | | | | |
|---|---|---|---|---|
| Here, Residual ratio (%)* = [(Weight of water insoluble solid) / (Weight of untreated beans)] × 100 (n = 4) | | | | |

## Claims

1. A method of producing polysaccharides from coffee beans or/and a coffee extraction residue, comprising:
(a) step of milling coffee beans or/and a coffee extraction residue to the particle which diameter is from 10.0 µm to 5.0 mm,
(b1) step of conducting heat treatment at 50 to 100°C in the presence of diluted alkali, after the step of (a),
(c1) step of treating with cellulase, after the step of (b1),
(b2) step of conducting heat treatment at 120°C or more in the presence of diluted alkali, after the step of (c1), and
(c2) step of treating with cellulase, after the step of (b2).

2. The method of producing polysaccharides from coffee beans or/and a coffee extraction residue according to Claim 1, wherein 85% or more of the cell wall component of coffee beans and/or a coffee extraction residue is solubilized.

3. The method of producing polysaccharides from coffee beans or/ and a coffee extraction residue according to Claim 1 or 2, wherein the cell wall component is composed of arabinogalactan and galactan to form a cell wall structure.

4. The method of producing polysaccharides from coffee beans or/and a coffee extraction residue according to Claim 1 to 3, wherein the polysaccharides include arabinogalactan or galactan.

5. The method of producing polysaccharides from coffee beans or/and a coffee extraction residue according to Claim 1 to 4, wherein the polysaccharides include galactomannan containing 30% or more of galactose.
